# EUROPÄISCHE PATENTSCHRIFT

(11) **EP 3 960 194 B1**
(45) Veröffentlichungstag und Bekanntmachung des Hinweises auf die Patenterteilung: **20.03.2024**
(21) Anmeldenummer: 21194431.9
(22) Anmeldetag: 01.09.2021
(51) Int. Cl.: A61K 36/534, A61K 36/82, A61K 36/906, A61K 35/644, A61K 36/752, A61K 36/9068, A61P 13/10

(54) **ZUSAMMENSETZUNG ZUR VERMINDERUNG DER SYMPTOME DER NYKTURIE**
COMPOSITION FOR REDUCING THE SYMPTOMS OF NOCTURIA
COMPOSITION DESTINÉE À LA RÉDUCTION DES SYMPTÔMES DE NYCTURIE

(30) Priorität: 01.09.2020 DE 102020122873
(43) Veröffentlichungstag der Anmeldung: 02.03.2022
(73) Patentinhaber: Loos, Volker, 78532 Tuttlingen (DE)
(72) Erfinder: Loos, Volker, 78532 Tuttlingen (DE)
(74) Vertreter: Stolmár & Partner Patentanwälte PartG mbB

(56) Entgegenhaltungen:
- EP-A1- 3 571 933
- EP-B1- 1 824 450
- DATABASE WPI Week 202006 Thomson Scientific, London, GB; AN 2020-65547A XP002805246, & CN 111 363 622 A (UNIV JILIN) 3. Juli 2020 (2020-07-03)
- DATABASE WPI Week 201904 Thomson Scientific, London, GB; AN 2018-94456F XP002805247, & CN 108 835 601 A (FUYANG FUMENG FOOD CO LTD) 20. November 2018 (2018-11-20)

## Beschreibung

### Gebiet der Erfindung

Die Erfindung betrifft eine Zusammensetzung umfassend Minze, grünen Tee, Ingwer und gegebenenfalls Frucht- oder Gemüsesaft aus einer Frucht- oder Gemüseart oder mehreren Frucht- oder Gemüsearten und Wasser, ein Verfahren zur Herstellung dieser Zusammensetzung sowie die Verwendung dieser Zusammensetzung zur Verminderung der Symptome der Nykturie, nämlich der Verminderung des nächtlichen Harndrangs einer Person.

### Stand der Technik

Als Nykturie wird die mehrmalige Blasenentleerung während der Nachtschlafphase bezeichnet. Betroffene, vor allem Menschen ab 50 Jahren, wobei Frauen und Männer gleich stark betroffen sind, wachen während der Schlafphase aufgrund von Harndrang auf und müssen die Blase entleeren, sodass mehrfache Unterbrechungen des Nachtschlafes auftreten. Von einer Nykturie spricht man üblicherweise erst, wenn die Person in einer Nacht mehr als zweimal Wasser lassen muss. Häufig gehen mit einer Nykturie auch Schmerzen beim Wasserlassen einher.

Das nächtliche Entleeren der Blase ist eine der häufigsten Ursachen für Schlafstörungen und Schlafunterbrechungen, die wiederum zu Müdigkeit, Kopfschmerzen, Konzentrationsstörungen und Depression führen können. Die *International Continence Society* (ICS) stuft die Nykturie sogar als klinisch bedeutsames Problem ein, da Lebensqualität und auch Leistungsfähigkeit der Betroffenen aufgrund der Unterbrechungen ihres Schlafes signifikant eingeschränkt sind.

Zur Linderung des nächtlichen Harndrangs sind verschiedene schulmedizinische Therapieansätze bekannt. Desmopressin, ein synthetisches Analogon des körpereigenen antidiuretischen Peptidhormons (ADH) Vasopressin, fördert die Rückresorption von Wasser und kann daher zur Verminderung des nächtlichen Harndrangs verabreicht werden. Aufgrund der Pharmakodynamik, die zu einer Volumenvermehrung mit relativem Elektrolytmangel führt, treten allerdings häufig teilweise starke Nebenwirkungen auf, wie Kopfschmerzen, Übelkeit, Erbrechen, Gewichtszunahme und teilweise sogar Hirnödeme, die mit Krampfanfällen beziehungsweise Bewusstseinseinschränkungen verbunden sein können. Des Weiteren gibt es selten Nebenwirkungen wie Allergien und Herzkreislaufprobleme (beispielsweise Angina Pectoris), die mit dem Blutdruckanstieg einhergehen.

Eine Nykturie ist häufig Symptom einer Grunderkrankung, wie einer Zystitis, einer benignen Prostatahyperplasie, einer Niereninsuffizienz und Diabetes mellitus.

Eine Zystitis ist eine entzündliche Erkrankung der Harnblase. Sie zählt wie die Urethritis (Harnröhrenentzündung) und Urozystitis (gemeinsame Entzündung von Harnblase und Harnröhre) zu den Infektionen der ableitenden Harnwege beziehungsweise unteren Harnwegsinfektionen. Als obere Harnwegsinfektion wird die Pyelonephritis bezeichnet. Symptome einer Zystitis sind unter anderem Schmerzen und Brennen beim Wasserlassen, häufiger Harndrang mit geringen Urinportionen sowie Blasenkrämpfe.

Die schulmedizinische Behandlung einer Zystitis erfolgt in der Regel mit der Gabe von Antibiotika, wodurch Nebenwirkungen, wie insbesondere eine Störung der Darmflora verursacht werden.

Eine benigne Prostatahyperplasie ist eine gutartige Vergrößerung der Prostata, die zu unangenehmen Beschwerden beim Wasserlassen führt.

Die schulmedizinische medikamentöse Behandlung einer benignen Prostatahyperplasie erfolgt in der Regel mit α-Blockern, die gleichzeitig das Herz-Kreislauf-System negativ beeinflussen und so zu Schwindel, Kopfschmerzen und Abgeschlagenheit führen können, 5-α-Reduktasehemmern, die zu Libidoverlust und Impotenz führen können, Phosphodiesterasehemmer, die zu Kopf- und Gliederschmerzen führen können, oder Anticholinergika, die zu einer erhöhten Herzfrequenz, erhöhtem Blutdruck und erhöhtem Augeninnendruck führen können.

Eine Niereninsuffizienz bedeutet, dass die Niere ihre Funktion Abbaustoffe auszuscheiden, die beim Stoffwechsel entstehen und mit dem Harn ausgeschieden werden müssen, in der Regel irreversibel verliert. Die häufigsten Ursachen für eine Niereninsuffizienz sind Diabetes mellitus und Bluthochdruck.

Die schulmedizinische Behandlung einer Niereninsuffizienz erfolgt in der Regel mittels reichlicher Flüssigkeitszufuhr in Kombination mit Diuretika. Dadurch werden die Symptome der Nykturie allerdings noch weiter verstärkt. Weiterhin können die die Niereninsuffzienz begünstigenden Erkrankungen behandelt werden, beispielsweise mittels ACE-Hemmern, die allerdings die Niere zusätzlich belasten und das Problem mittelfristig verstärken, und AT1-Blockern gegen den Bluthochdruck, die Kopfschmerzen und Schwindel verursachen können, oder mittels Lipidsenkern zur Senkung des Gehalts an Blutfetten, die wiederum Verdauungsstörungen, Kopf- und Gliederschmerzen und Überempfindlichkeitsreaktionen verursachen können.

Diabetes mellitus, auch als Zuckerkrankheit bezeichnet, ist ein Überbegriff für verschiedene Störungen des Stoffwechsels. Allen gemeinsam ist, dass sie zu erhöhten Blutzuckerwerten führen, weil die Patientinnen und Patienten einen Insulinmangel und/oder eine Insulinresistenz haben.

Durch Behandlung der Grunderkrankung kann häufig auch das Symptom der Nykturie vermindert werden.

Des Weiteren können eine Blaseninkontinenz, eine Herzinsuffizient, eine Schwangerschaft und harntreibende Medikamente, wie beispielsweise Diuretika ursächlich für eine Nykturie sein.

Häufig überlagern sich mehrere Ursachen für eine Nykturie beziehungsweise bestehen nebeneinander und verstärken sich gegenseitig.

Auch verschiedene pflanzliche Wirkstoffe werden zur Linderung der Symptome der Nykturie angewendet, beispielsweise Kürbiskernöl, Kürbissamenöl, Extrakte aus Sägepalmenfrüchten und Brennnesselwurzeln, deren Wirkung allerdings nur gering ist.

Zahlreiche weitere pflanzliche Wirkstoffe sind bekannt, die positive Eigenschaften auf den menschlichen Körper haben, jedoch bisher nicht zur Behandlung der Nykturie eingesetzt werden.

Ingwer ist reich an Vitamin C und enthält darüber hinaus Magnesium, Eisen, Kalzium, Kalium, Natrium und Phosphor.

Zubereitungen aus dem Ingwerrhizom werden antibakterielle, antioxidative, antiemetische, entzündungshemmende, immunsystemstärkende sowie anregende Effekte auf die Magensaft-, Speichel- und Gallenbildung sowie die Darmfunktion beziehungsweise die Darmflora zugesprochen. Diese werden daher beispielsweise zur Behandlung von Rheuma, Muskelschmerzen oder Erkältungen angewendet.

Ingwer enthält Shogaol, das eine zytotoxische Wirkung auf Tumorstammzellen aufweist, die verantwortlich für Wachstum, Resistenz und Metastasierung sind. Daher hat Ingwer eine krebshemmende Wirkung.

Ingwer enthält außerdem Gingerol, das die Schmerzrezeptoren beeinflusst und daher schmerzlindernd wirken kann.

In DE 20 2009 004 878 U1 und DE 102008002999 A1 werden Zusammensetzungen offenbart, die Ingwer und Kamille enthalten und unter anderem als kosmetisches Mittel, insbesondere als Haarwuchs- und Nagelpflegemittel, als Nahrungsergänzungsmittel oder Allzweckreiniger verwendet werden.

Grüner Tee ist für seine vielfältigen positiven Effekte auf den Körper bekannt, unter anderem für seine blutdrucksenkende, immunsystemstärkende und blutzuckersenkende Wirkung. Grüner Tee verringert außerdem das Risiko an Krebs zu erkranken und wirkt gegen Diabetes mellitus.

EP 2 066 186 B1 offenbart einen Extrakt aus grünem Tee, der als funktionelles Lebensmittel oder Nahrungsergänzungsmittel verwendet wird.

EP 3 597 047 A1 offenbart Schokolade, die pulverförmigen grünen Tee enthält.

Wirkstoffe der Minze sind insbesondere deren ätherisches Öl, Labiatengerbstoffe und Flavonoide. Als Tee verabreichte Minze wirkt krampflösend und verdauungsfördernd bei Beschwerden im Magen-Darm-Bereich sowie beruhigend und schlaffördernd bei Unruhezuständen. Das ätherische Öl der Minze wird auch zum Einreiben bei Migräne, Kopf- und Nervenschmerzen sowie zum Inhalieren bei Erkältungskrankheiten verwendet. Minze wirkt außerdem schmerzlindernd.

DE 10 065 832 A1 offenbart zudem ein Mittel zur Gewichtsreduktion auf der Basis von Minze, Melisse und Fenchel.

Zitronensaft hydriert, fördert und verbessert die Verdauung, stärkt das Immunsystem, reinigt die Nieren, schützt Gelenke, entgiftet, entsäuert und hat eine unterstützende Wirkung bei der Gewichtsreduktion im Rahmen einer Diät.

Zitrone ist reich an basischen Bestandteilen, wie Kalium und Magnesium, arm an säurebildenden Aminosäuren, enthält Antioxidantien, Vitamin C und aktivierende Fruchtsäuren, regt die körpereigene Basenbildung an, indem die Bildung von basischer Galle in der Leber gefördert wird, verhindert die Bildung von Schlacken, wirkt entzündungshemmend, ist wasserreich und hilft daher bei der Ausschwemmung von Schlacken und fördert die Verdauung sowie Regeneration von Schleimhäuten.

Auch als Lebensmittelzusatz wird Zitrone verwendet. DE 20 2007 010 105 U1 offenbart den Zusatz von Zitronenpulver zu Schokolade, wodurch eine geschmacklich besonders ansprechende Variante bereitgestellt werden soll.

Die EP 1 824 450 B1 offenbart pharmazeutische Zusammensetzungen zur Verabreichung an den Nasaltrakt, insbesondere Trockenpulverzusammensetzungen, die Cellulose, insbesondere Hyroxypropylmethylcellulose (HPMC), und einen oder mehrere therapeutische Wirkstoffe umfassen. Es hat sich gezeigt, dass die Verabreichung von HPMC in die Nasenhöhle die natürliche Schleimbildung fördern kann.

Die EP 3 571 933 A1 offenbart eine süßende oder gesüßte Zusammensetzung, die Saccharose, Rubusosid oder ein Rubusosid-Derivat und ein Tannin umfasst. Ferner wird die Verwendung einer solchen Zusammensetzung zum Süßen von Getränken sowie zur Herstellung eines Sirups als Vorstufe bei der Zubereitung von Getränken und auch als Aromastoff mit modifizierenden Eigenschaften und zur Verringerung des Saccharosegehalts in Getränken, ohne im Wesentlichen ein verringertes Süßeempfinden aufzuweisen, offenbart. Des Weiteren wird die Verwendung einer Kombination offenbart, die Rubusosid oder ein Rubusosid-Derivat und ein Tannin umfasst, um die anhaltende Süße von künstlichen Süßungsmitteln zu verkürzen und das Einsetzen der zeitlichen Süßeempfindung dieses künstlichen Süßungsmittels in Richtung derjenigen von natürlichen Zuckern zu verschieben.

Honig besteht, zu ungefähr 80 Prozent aus Zucker und zu ungefähr 20 Prozent aus Wasser. Darüber hinaus enthält Honig einige Vitamine, wie Vitamin C, Thiamin, Riboflavin und Niacin, und Mineralstoffe, wie Kalzium, Kalium, Natrium und Chlor. Zudem finden sich in Honig Enzyme, die entzündungshemmend wirken, sowie Aminosäuren und Spurenelemente wie Eisen, Zink und Kupfer. Honig wirkt darüber hinaus antibakteriell und wundheilungsfördernd.

Vor diesem Hintergrund ist die Entwicklung einer Zusammensetzung aus biologischen Wirkstoffen erforderlich, die eine Linderung der Symptome der Nykturie, nämlich eine Verminderung des nächtlichen Harndrangs erreicht, ohne unerwünschte Nebenwirkungen hervorzurufen.

### Darstellung der Erfindung

Aufgabe der vorliegenden Erfindung ist es daher, eine Zusammensetzung sowie ein Verfahren zur Herstellung dieser Zusammensetzung bereitzustellen, die den nächtlichen Harndrang vermindert, ohne dabei die Gesundheit beeinträchtigende Nebenwirkungen hervorzurufen.

Die erfindungsgemäße Zusammensetzung zur Verwendung bei der Verringerung von Nykturiesymptomen umfasst Minze, grünen Tee, Ingwer und gegebenenfalls Frucht- oder Gemüsesaft aus einer Frucht- oder Gemüseart oder mehreren Frucht- oder Gemüsearten und Wasser.

Bevorzugt umfasst die Zusammensetzung getrocknete Minzeblätter, frische Minzeblätter, getrocknete Blätter grünen Tees, frischen Ingwer und Zitrusfruchtsaft aus einer Fruchtart oder mehreren Fruchtarten.

Bevorzugt umfasst die Zusammensetzung getrocknete Minzeblätter, frische Minzeblätter, getrocknete Blätter grünen Tees, frischen Ingwer und Zitronensaft.

Der Begriff "Minze" gemäß der vorliegenden Erfindung bezeichnet Heil- und Gewürzpflanzen der Pflanzengattung der Familie der Lippenblütengewächse (*Lamiaceae*). Die meisten der etwa 20 bis 30 Arten sind in den gemäßigten Gebieten der Nordhalbkugel beheimatet und gedeihen vorwiegend an feuchten Standorten.

Die exakte Abgrenzung der Arten ist nicht möglich, da Minzen dazu neigen, natürliche Hybriden (Bastarde) zu bilden.

Als "Minze" gemäß der vorliegenden Erfindung kann jede Pflanze aus der Gattung der Minzen (Mentha) innerhalb der Familie der Lippenblütengewächse (Lamiaceae) verwendet werden, ausgewählt aus der Gruppe umfassend *Mentha piperita, Mentha aquatica, Mentha spicata, Mentha rotundifolia, Mentha longifolia, Mentha arvensis, Mentha cervina, Mentha gentilis, Mentha pulegium, Mentha cordifolia, Mentha crispa, Mentha rosanii, Mentha viridis, Mentha suaveolens, Mentha macrostachya, Mentha Citrata, Mentha nemorosa, Mentha requienii, Mentha alaica, Mentha asiatica, Mentha gattefossei,* verschiedene Unterarten und Varietäten der vorgenannten, sowie Hybride.

Der Begriff "getrocknete Minzeblätter" gemäß der vorliegenden Erfindung bezeichnet Minzeblätter, denen durch Trocknung Wasser entzogen wurde. Gemäß der vorliegenden Erfindung können als getrocknete Minzeblätter lose Minzeblätter verwendet werden, die aus ganzen und/oder gebrochenen Blättern und/oder Stielen der Minzepflanze bestehen. Die Minzeblätter und/oder Stiele können zerdrückt, gerieben, gemahlen oder pulverisiert vorliegen. Ebenso kann portionierter Tee verwendet werden, der in Beuteln, Pads, Kapseln oder Teesticks abgepackt ist.

Getrocknete Minzeblätter bieten den Vorteil, dass sie länger haltbar sind. Dadurch kann die Herstellung der erfindungsgemäßen Zusammensetzung vereinfacht werden.

Der Begriff "frische Minzeblätter" gemäß der vorliegenden Erfindung bezeichnet Minzeblätter, die gegebenenfalls kurz vor dem Aufkochen von einer Minzepflanze geerntet wurden. Bevorzugt werden die Minzeblätter verwendet. Es ist jedoch auch möglich die Stiele der Minzepflanze zu verwenden.

Frische Minzeblätter bieten den Vorteil, dass sie in hoher Konzentration Wirkstoffe, wie das ätherische Öl der Minze, Labiatengerbstoffe und Flavonoide enthalten.

Die erfindungsgemäße Verwendung von Minze führt dazu, dass die Harnblase aufgrund der krampflösenden Wirkung der Minze dehnbarer wird und dementsprechend ein größeres Harnvolumen fassen kann. Dadurch können wiederum die Symptome der Nykturie verringert werden können.

Die erfindungsgemäße Verwendung von Minze führt außerdem dazu, dass Schmerzen beim Wasserlassen aufgrund der schmerzlindernden Wirkung der Minze verringert werden können.

Der Begriff "grüner Tee" gemäß der vorliegenden Erfindung bezeichnet Pflanzenteile der Teepflanze (*Camellia sinensis*) aus der Gattung der Kamelien (*Camellia*) innerhalb der Familie der Teestrauchgewächse (*Theaceae*). Der Begriff "grüner Tee" gemäß der vorliegenden Erfindung umfasst auch Matcha, ein zu Pulver vermahlener grüner Tee, der von Teesträuchern geerntet wird, die vor der Ernte beschattet werden. Nach der Ernte werden die Teeblätter gedämpft, getrocknet und das Blattfleisch, Tencha genannt, nach Entfernung aller groben Blattgefäße zu Pulver gemahlen.

Der Begriff "getrocknete Blätter grünen Tees" gemäß der vorliegenden Erfindung bezeichnet Pflanzenteile der Teepflanze (*Camellia sinensis*) aus der Gattung der Kamelien (*Camellia*) innerhalb der Familie der Teestrauchgewächse (*Theaceae*)*,* hauptsächlich Blätter, die nach dem Welken der frisch gepflückten Blätter erhitzt, geröstet oder gedämpft wurden, aber im Gegensatz zu schwarzem Tee nicht fermentiert werden.

Gemäß der vorliegenden Erfindung können als getrocknete Blätter grünen Tees lose Pflanzenteile der Teepflanze Camellia sinensis verwendet werden, die aus ganzen und gebrochenen Blättern und Knospen, aber auch Stielen bestehen oder zu Pulver gemahlen sind. Ebenso kann portionierter Tee verwendet werden, der in Beuteln, Pads, Kapseln oder Teesticks abgepackt ist.

Getrocknete Blätter grünen Tees bieten den Vorteil, dass sie länger haltbar sind. Dadurch kann die Herstellung der erfindungsgemäßen Zusammensetzung vereinfacht werden.

Es können auch frische Blätter grünen Tees verwendet werden, die den Vorteil bieten, dass sie in hoher Konzentration Wirkstoffe enthalten. Dadurch kann die Wirkung der Zusammensetzung verstärkt werden
Die erfindungsgemäße Verwendung von grünem Tee führt dazu, dass eine der Nykturie potentiell zugrundeliegende benigne Prostatahyperplasie aufgrund der das Krebsrisiko verringernden Wirkung des grünen Tees verringert werden kann. Auch eine der Nykturie potentiell zugrundeliegende Niereninsuffizienz kann mittels grünem Tee verringert werden, da grüner Tee sowohl blutdrucksenkend als auch gegen das die Niereninsuffizienz verstärkende Diabetes mellitus wirkt. Dadurch können wiederum die Symptome der Nykturie verringert werden.

Der Begriff "Ingwer", auch Ingber, Imber, Immerwurzel oder Ingwerwurzel genannt, gemäß der vorliegenden Erfindung bezeichnet das Ingwerrhizom (auch Ingwerwurzelstock genannt), das unterirdisch oder dicht über dem Boden wachsende Sprossachsensystem des Ingwers, aus der Gattung Ingwer (*Zingiber*) innerhalb der Familie der Ingwergewächse (*Zingiberaceae*)*.* Gemäß der vorliegenden Erfindung kann frischer und/oder getrockneter Ingwer verwendet werden.

Der Begriff "frischer Ingwer" gemäß der vorliegenden Erfindung bezeichnet geerntete und gewaschene Ingwerrhizome, die nicht getrocknet und/oder pulverisiert sind.

Der Begriff "getrockneter Ingwer" gemäß der vorliegenden Erfindung bezeichnet Ingwerrhizome, denen durch Trocknung Wasser entzogen wurden, sodass der Wassergehalt zwischen 15 und 25 Prozent liegt. Er kann zerdrückt, gerieben, gemahlen oder pulverisiert vorliegen.

Die erfindungsgemäße Verwendung von Ingwer führt dazu, dass eine der Nykturie potentiell zugrundeliegende Zystitis mittels der entzündungshemmenden Wirkung des Ingwers verringert werden kann, wodurch wiederum die Symptome der Nykturie verringert werden können.

Die erfindungsgemäße Verwendung von Ingwer führt außerdem dazu, dass Schmerzen beim Wasserlassen aufgrund der schmerzlindernden Wirkung insbesondere des im Ingwer enthaltenen Gingerols verringert werden können.

Der Begriff "Frucht- oder Gemüsesaft" bezeichnet ein aus einer oder mehreren Frucht- und Gemüsearten gewonnenes flüssiges Erzeugnis, das für die menschliche Ernährung bestimmt ist, insbesondere verschiedene Direktsäfte, Säfte aus Konzentrat, Nektare aus Obst und Gemüse. Der erfindungsgemäße Frucht- oder Gemüsesaft kann beliebig aus einer Frucht- oder Gemüseart oder mehreren Frucht- und/oder Gemüsearten hergestellt sein, ausgewählt aus der Gruppe umfassend Aroniabeeren, Kranbeeren (Cranberry), Johannisbeeren, Mango, Granatäpfeln, Kirschen, Äpfeln, Orangen, Grapefruits, Bananen, Maracuja, Erdbeeren, Himbeeren, Brombeeren, Heidelbeeren, Papaya, Trauben, Sanddorn, Ananas, Holunderbeere, Quitte, Pfirsich, Weißdorn, Spinat, Mangold, Rhabarber, Rote Beete, Schlehe, Passionsfrucht, Sellerie, Sauerkraut, Gurke, Karotte, Tomate, Grünkohl, Gerstengrassaft und Weizengrassaft.

Das Mischungsverhältnis sowie die Frucht beziehungsweise Gemüsesorte/n ist/sind dabei nach Geschmack und Verträglichkeit beliebig wählbar und trägt/tragen zur geschmacklichen Verbesserung der erfindungsgemäßen Zusammensetzung bei.

Der Begriff "Zitrusfrucht" gemäß der vorliegenden Erfindung bezeichnet jede Frucht einer Pflanze aus der Gattung der Zitruspflanzen (*Citrus*) aus der Familie der Rautengewächse (*Rutaceae*). Zitrusfrüchte gemäß der vorliegenden Erfindung sind ausgewählt aus der Gruppe umfassend Mandarine (*Citrus reticulata*), Clementine (*Citrus *aurantium, Citrus clementina*), Satsuma (*Citrus *aurantium, Citrus unshiu*), Apfelsine, auch Orange genannt, (*Citrus *aurantium, Citrus sinensis*), Bitterorange, auch Pomeranze genannt, (*Citrus *aurantium*), Bergamotte (*Citrus* ×*limon, Citrus bergamia*), Pampelmuse, auch Pomelo genannt (*Citrus maxima*), Grapefruit (*Citrus *aurantium, Citrus paradisi*)*,* Echte Limette (*Citrus* ×*aurantiifolia*), Gewöhnliche Limette (*Citrus* ×*aurantiifolia, Citrus latifolia*), Kaffernlimette, auch Kaffirlimette oder Mauritiuspapeda genannt, (*Citrus hystrix*), Rangpurlimette (*Citrus* ×*jambhiri*), Kumquats (*Citrus japonica, Fortunella*) Zitronatzitrone (*Citrus medica*), Meyerzitrone (*Citrus* × *Meyeri, Citrus* × *limon* meyeri beziehungsweise *Citrus* ×*jambhin* Meyer), besonders bevorzugt ausgewählt aus der Gruppe der Zitronen (*Citrus* ×*limon*) umfassend die Sorten Zagara Bianca, Lunario, Feminello, Santa Teresa, Primofiori, Verna oder Berna, Interdonato, Feminello, Lisbon, Eureka, Eureka Rosa, Citrus Limetta, Sfusato Amalfitano, Bianchetto, Lipo, Nine Pounder, Citrus Volkameriana (Volkamer Zitrone).

Der Begriff "Zitrusfruchtsaft" gemäß der vorliegenden Erfindung bezeichnet ein aus Zitrusfrüchten einer oder mehrerer Fruchtarten gewonnenes flüssiges Erzeugnis, das für die menschliche Ernährung bestimmt ist. Bevorzugt handelt es sich bei dem Zitrussaft um Zitronensaft.

Die erfindungsgemäße Verwendung von Zitronensaft führt dazu, dass aufgrund der entzündungshemmenden Wirkung des Zitronensaftes eine der Nykturie potentiell zugrundeliegende Zystitis zusätzlich verringert werden kann, wodurch wiederum die Symptome der Nykturie verringert werden können.

Des Weiteren kann die Verwendung von Zitronensaft zur geschmacklichen Verbesserung der erfindungsgemäßen Zusammensetzung beitragen.

Mit der gemeinsamen Verwendung der unterschiedlichen Zutaten, nämlich Minze, grüner Tee, Ingwer und Zitronensaft können die verschiedenen und sich teilweise überlagernden und verstärkenden Ursachen für eine Nykturie effektiv bekämpft werden. Dabei kann sich die Wirkung der unterschiedlichen Zutaten gegenseitig begünstigen und so zu einer synergistischen Gesamtwirkung der erfindungsgemäßen Zusammensetzung führen.

Bevorzugt umfasst die Zusammensetzung ferner weiteren Tee. Der Begriff "weiterer Tee" gemäß der vorliegenden Erfindung umfasst verschiedene Teesorten wie schwarzer Tee, Weißer Tee, Oolong Tee, Zealong Tee, Aromatisierter Tee, Rotbuschtee (Rooibostee), Honigbuschtee (Honeybushtee), Früchtetee, Kräutertee, Ayurvedatee, Flugtee, Eistee, sowie Mischungen davon. Das Mischungsverhältnis sowie die Teesorte/n ist/sind dabei nach Geschmack und Verträglichkeit beliebig wählbar und tragen zur geschmacklichen Verbesserung der erfindungsgemäßen Zusammensetzung bei.

Ferner ist die Verwendung von Minze, grünem Tee, Ingwer, Frucht- oder Gemüsesaft und weiterem Tee in Bioqualität bevorzugt, das heißt aus ökologisch kontrolliertem Anbau stammend, nicht gentechnisch verändert und ohne Einsatz von chemisch-synthetischen Pflanzenschutzmitteln, Kunstdünger oder Klärschlamm angebaut.

Durch die Verwendung von Zutaten in Bioqualität kann die Aufnahme von Schadstoffen verringert werden, die in konventionell angebauten Lebensmitteln in hohem Maße vorkommen können.

Bevorzugt umfasst die Zusammensetzung 2-25 g getrocknete Minzeblätter, 5-30 g frische Minzeblätter, 1,5-20,0 g getrocknete Blätter grünen Tees, 40-400 g frischen Ingwer, 400-2000 ml Wasser und 10-2000 ml Frucht- oder Gemüsesaft.

Je nach gewünschter Viskosität, also Zähflüssigkeit der Zusammensetzung kann in Bezug auf die festen Bestandteile, nämlich getrocknete Minzeblätter, frische Minzeblätter, getrocknete Blätter grünen Tees und Ingwer eine entsprechende Menge an flüssigen Zutaten, nämlich Wasser und Zitronensaft gewählt werden. Je mehr Wasser und/oder Zitronensaft die Zusammensetzung enthält, desto dünnflüssiger ist sie. Eine dünnflüssige Zusammensetzung bietet Vorteile hinsichtlich einer guten Trinkbarkeit, eines zurückhaltenden Geschmacks und einer einfachen Dosierbarkeit (besseres Ausflussverhalten aus dem Aufbewahrungsbehältnis). Eine dickflüssige Zusammensetzung bietet Vorteile hinsichtlich einer hohen Konzentration an Wirkstoffen und eines intensiven Geschmacks.

Bevorzugt umfasst die Zusammensetzung zusätzlich getrockneten Ingwer.

Bevorzugt umfasst die Zusammensetzung zusätzlich 2,5-15,0 g getrockneten Ingwer, bevorzugt 5-10 g getrockneten Ingwer, besonders bevorzugt 7,5 g getrockneten Ingwer.

Getrockneter Ingwer bietet den Vorteil, dass er länger haltbar ist. Dadurch kann die Herstellung der erfindungsgemäßen Zusammensetzung vereinfacht werden.

Bevorzugt umfasst die Zusammensetzung ferner 10-200 ml Honig, insbesondere 20-150 ml Honig und besonders bevorzugt 30-100 ml Honig.

Die erfindungsgemäße Verwendung von Honig führt dazu, dass eine der Nykturie potentiell zugrundeliegende Zystitis mittels der entzündungshemmenden Wirkung des Honigs verringert werden kann, wodurch wiederum die Symptome der Nykturie verringert werden können. Bei einer bakteriell bedingten Zystitis können darüber hinaus die schädlichen Bakterien aufgrund der antibakteriellen Wirkung des Honigs direkt bekämpft werden.

Des Weiteren kann die Verwendung von Honig zur geschmacklichen Verbesserung der erfindungsgemäßen Zusammensetzung beitragen.

Die Erfindung ist auch auf eine orale Darreichungsform zur Verwendung bei der Verringerung von Nykturiesymptomen umfassend die erfindungsgemäße Zusammensetzung in Form einer Tablette, einer Kapsel oder eines Granulats gerichtet.

Bevorzugt umfasst die Zusammensetzung der oralen Darreichungsform ferner einen Füllstoff aus der Gruppe von Lactose, Cellulose, Stärke, Saccharose, Polyethylenglykol und Polyethylenoxid oder aus einer Kombination der vorgenannten Stoffe.

Durch die zusätzliche Komponente des Füllstoffes kann die Konsistenz der Zusammensetzung derart eingestellt werden, dass diese zur Herstellung einer Tablette verwendbar ist.

Vorzugsweise liegt der Füllstoff in mikrokristalliner Form vor. Damit kann die Mischbarkeit der einzelnen Komponenten der Zusammensetzung verbessert werden.

Vorzugsweise umfasst die Zusammensetzung ferner einen Emulgator und/oder ein Verdickungs- und Bindemittel und/oder ein Umhüllungsmittel und/oder ein Antioxidans und/oder einen Konservierungsstoff und/oder ein Süßungsmittel und/oder einen Geschmackskorrigienten und/oder ein Fettpulver.

Mittels des Emulgators können gegebenenfalls nicht mischbare Komponenten der Zusammensetzung vermengt und stabilisiert werden.

Mittels des Verdickungs- und Bindemittels kann der Zusammenhalt der als Tablette ausgeformten Zusammensetzung verbessert werden.

Mittels des Umhüllungsmittels kann die als Tablette ausgeformte Zusammensetzung vor ungewolltem Zerfall sowie vor schädigenden äußeren Einflüssen geschützt werden.

Mittels des Antioxidans kann die Oxidation der Zusammensetzung verlangsamt beziehungsweise verhindert werden, wodurch die Haltbarkeit der Zusammensetzung erhöht werden kann.

Mittels des Konservierungsstoffes kann die Haltbarkeit der Zusammensetzung verbessert werden.

Mittels des Süßungsmittels kann der Geschmack der Zusammensetzung verbessert werden.

Mittels des Geschmackskorrigienten kann ebenfalls der Geschmack der Zusammensetzung verbessert werden.

Mittels des Fettpulvers kann die Herstellbarkeit einer Tablette auf Basis der Zusammensetzung verbessert werden. Außerdem kann mittels des Fettpulvers der Zusammenhalt der als Tablette ausgeformten Zusammensetzung verbessert werden.

Bevorzugt wird beziehungsweise wurde die Zusammensetzung der oralen Darreichungsform gefriergetrocknet.

Dadurch kann ein Granulat erhalten werden. Ein Granulat im Sinne der Erfindung Erfindungsgemäß ist das Granulat ein pulverförmiges, feinkörniges Trockenprodukt auf Basis der erfindungsgemäßen Zusammensetzung. Dieses kann neben dem Gefriertrocknen auch mittels anderer Trocknungsverfahren hergestellt sein.

Das so erhaltene Pulver oder Granulat kann zur Herstellung von Lösungen, Emulsionen, Tropfen und Suspensionen zur Einnahme verwendet werden. Ebenso kann es zur Herstellung von Kapseln, wie Hartkapseln, Weichkapseln oder Flüssigkapseln sowie von Tabletten, wie nicht überzogene Tabletten, überzogene Tabletten, Brausetabletten, Tabletten zur Herstellung einer Lösung zur Einnahme, Schmelztabletten oder Kautabletten verwendet werden.

Die Herstellung der erfindungsgemäßen Zusammensetzung umfasst folgende Schritte:
a) Bereitstellen von Minze, grünem Tee, Ingwer und gegebenenfalls Frucht- oder Gemüsesaft aus einer Frucht- oder Gemüseart oder mehreren Frucht- oder Gemüsearten und Wasser;
b) Zerkleinern des Ingwers;
c) Aufkochen des Ingwers gegebenenfalls in Wasser, Abkühlen und 3 min bis 48 h ziehen lassen;
d) Aufkochen der Mischung aus Minze und grünem Tee gegebenenfalls in Wasser, Abkühlen und 3 min bis 48 h ziehen lassen;
e) Jeweils Abtrennen der Flüssigkeit aus Schritt c) und d) von den Feststoffen;
f) Mischen der Flüssigkeiten aus Schritt c) und d);
g) 3 bis 5 Tage ziehen lassen der Flüssigkeit aus Schritt f) bei Temperaturen von 0-10 °C; und
h) Gegebenenfalls Mischen der Flüssigkeit aus Schritt g) mit Frucht- oder Gemüsesaft aus einer Frucht- oder Gemüseart oder mehreren Frucht- oder Gemüsearten.

Bevorzugt wird während Schritt a) Ingwer ohne Schale bereitgestellt. Dadurch kann der Ingwer besser zerkleinert werden. Es kann aber auch ungeschälter Ingwer bereitgestellt werden.

Um Schritt b) durchzuführen, kann beispielsweise ein Zerkleinerer verwendet werden oder ein beliebiges für die Verarbeitung von Lebensmitteln geeignetes Gerät, mit dem eine definierte Zerkleinerung von unterschiedlich großen Ingwerstücken mittels Schneiden und/oder Quetschen möglich ist. Diese können Küchenreiben, Sparschäler, Julienneschneider oder Messer sein. Der Ingwer kann damit beispielsweise halbiert, geviertelt, in Quader, Würfel, Polygone oder Scheiben geschnitten oder zerfasert werden.

Je länger während Schritt c) der gegebenenfalls in Wasser aufgekochte Ingwer gezogen lassen wird, desto mehr Wirkstoffe können aus den festen Bestandteilen des Ingwers in die Flüssigkeit übergehen. Je nach verwendetem Ingwer kann dabei nach kürzerer oder längerer Zeit ein Sättigungsgleichgewicht erreicht werden.

Bevorzugt wird unmittelbar nach Erreichen des Sättigungsgleichgewichts mit dem Herstellverfahren fortgefahren, um dessen Effektivität zu erhöhen.

Bevorzugt werden in Schritt c) die frischen und getrockneten Minzeblätter sowie die Blätter des grünen Tees jeweils getrennt voneinander aufgekocht.

Alternativ werden in Schritt c) die frischen und getrockneten Minzeblätter zusammen aufgekocht, während die Blätter des grünen Tees getrennt voneinander aufgekocht werden.

Der Begriff "Aufkochen" gemäß der vorliegenden Erfindung bezeichnet den Vorgang gegebenenfalls das Wasser mit Ingwer beziehungsweise mit Minze und grünem Tee für einen Zeitraum von 1 s bis 15 min sprudelnd zum Kochen zu bringen. Alternativ wird das Wasser sprudelnd zum Kochen gebracht und der Ingwer beziehungsweise die Blätter damit übergossen. Sprudelnd zum Kochen bringen bedeutet, dass das Wasser je nach Höhenlage eine Temperatur von ungefähr 100 °C aufweist beziehungsweise der Siedepunkt des Wassers erreicht wird.

Alternativ kann die Mischung aus Minze und grünem Tee und Wasser auf 60-90 °C erhitzt anstatt aufgekocht werden. Dadurch können gegebenenfalls hitzeempfindliche Wirkstoffe geschützt werden, wodurch wiederum die Wirkung der Zusammensetzung verbessert werden kann.

Je länger während Schritt d) die gegebenenfalls in Wasser aufgekochte Mischung aus Minze und grünem Tee gezogen lassen wird, desto mehr Wirkstoffe können aus den festen Bestandteilen der Minze und des grünen Tees in die Flüssigkeit übergehen. Je nach verwendeter Minze und verwendetem grünen Tee kann dabei nach kürzerer oder längerer Zeit ein Sättigungsgleichgewicht erreicht werden.

Bevorzugt wird unmittelbar nach Erreichen des Sättigungsgleichgewichts mit dem Herstellverfahren fortgefahren, um dessen Effektivität zu erhöhen.

Bevorzugt werden die Schritte c) und d) parallel ausgeführt. Dadurch kann die Effektivität des Herstellverfahrens erhöht werden.

Um Schritt e) durchzuführen, kann beispielsweise ein Sieb oder eine Zentrifuge verwendet werden.

Der Begriff "Abtrennen der Flüssigkeit von den Feststoffen" umfasst das Abgießen der Flüssigkeit, Dekantieren, Sieben und Filtern.

Bevorzugt werden die gemischten Flüssigkeiten während Schritt f) in die endgültigen Behältnisse gefüllt. Dadurch kann auf ein erneutes Umfüllen verzichtet werden, wodurch die Prozesseffizienz gesteigert werden kann.

Um Schritt g) durchzuführen, kann beispielsweise ein Kühlschrank verwendet werden.

Bevorzugt wird zur Herstellung der erfindungsgemäßen Zusammensetzung 40-400 g frischer Ingwer mit 200-800 ml Wasser 10-20 min aufgekocht. Zudem werden 2-25 g getrocknete Minzeblätter mit 5-30 g frischen Minzeblätter und 1,5-20,0 g getrockneten Blättern des grünen Tees mit 200-1200 ml Wasser 10-20 min aufgekocht.

Alternativ werden 200 g des zerkleinerten Ingwers mit 800 ml Wasser 10 min aufgekocht. Zudem werden 11,25 g getrocknete Minzeblätter mit 10 g frischen Minzeblättern und 8,75 g Blättern grünen Tees mit 1200 ml Wasser 10 min aufgekocht.

Alternativ werden 40 g des zerkleinerten Ingwers mit 800 ml Wasser 10 min aufgekocht. Zudem werden 2,25 g getrocknete Minzeblätter mit 6 g frischen Minzeblättern und 1,75 g Blättern grünen Tees mit 1200 ml Wasser 10 min aufgekocht.

Alternativ werden 400 g des zerkleinerten Ingwers mit 800 ml Wasser 10 min aufgekocht. Zudem werden 22,5 g getrocknete Minzeblätter mit 25 g frischen Minzeblättern und 17,5 g Blättern grünen Tees mit 1200 ml Wasser 10 min aufgekocht.

Dadurch können die oben beschriebenen verschiedenen bevorzugten Zusammensetzungen hergestellt werden.

Während Schritt h) kann das Mischungsverhältnis von Flüssigkeit aus Schritt g) mit Frucht- oder Gemüsesaft je nach Anwendungsfall gewählt werden. Bevorzugt beträgt das Mischungsverhältnis von Flüssigkeit aus Schritt g) mit Frucht- oder Gemüsesaft 4:1 oder 2:1 und besonders bevorzugt 1:1.

Bevorzugt wird während Schritt h) als Frucht- oder Gemüsesaft Zitronensaft verwendet.

Bevorzugt wird nach Schritt g), insbesondere während Schritt h) die Flüssigkeit ferner mit Honig gemischt.

Die erfindungsgemäße Zusammensetzung kann mit verschiedenen Zusätzen gemischt werden, um den Geschmack zu verbessern.

Zu diesen Zusätzen zählen unter anderem Süßstoff, Zucker, Stevia, Birkenzucker, Palmzucker oder Sirupen, wie Ahornsirup, Agavensirup, Birnendicksaft, Dattelsirup, Saft der Mannaesche, Melasse, Palmzuckersirup, Rübensirup oder Traubendicksaft. Das Mischungsverhältnis ist dabei nach Geschmack beliebig wählbar.

Die erfindungsgemäße Zusammensetzung kann mit verschiedenen pflanzlichen Ölen wie Kürbiskernöl, Kürbissamenöl, Mandelöl, Leinöl, Rapsöl, Hanföl, Olivenöl und/oder pflanzlichen Getränken wie Hafergetränke, Mandelgetränke, Sojagetränke, Getreidegetränke, Dinkelgetränke, Reisgetränke, Hanfgetränke, Lupinengetränke in einem je nach Anwendungsfall wählbaren Mischungsverhältnis gemischt werden. Bevorzugt beträgt das Mischungsverhältnis der erfindungsgemäßen Zusammensetzung damit 4:0,1; 2:0,1 oder 1:0,1.

Die Zusammensetzung kann mit weiterem Tee gemischt werden. Das Mischungsverhältnis ist nach Geschmack wählbar. Bevorzugt beträgt das Mischungsverhältnis der erfindungsgemäßen Zusammensetzung mit weiterem Tee 4:1; 2:1 oder 1:1.

Die Zusammensetzung kann mit Propolis, Algen, Matha, Ginseng, Acai, Aloe Vera, Guarana, Moringa, Chia, Flohsamenschalen, Sägepalmenfrüchten und/oder Brennnesselwurzeln sowie Extrakten davon gemischt werden.

Bevorzugt wird die erfindungsgemäße Zusammensetzung und/oder die erfindungsgemäße orale Darreichungsform zur Verminderung des nächtlichen Harndrangs verwendet. Dazu kann die erfindungsgemäße Zusammensetzung täglich oral eingenommen werden. Dies kann beispielsweise durch Trinken der Flüssigkeit oder durch Einnahme des Pulvers oder Granulats beziehungsweise der Tablette oder Kapsel geschehen.

In einer weiteren Ausführungsform werden täglich 40-200 ml der erfindungsgemäßen Zusammensetzung getrunken, bevorzugt 75-150 ml.

In einer besonders bevorzugten Ausführungsform werden täglich 50 ml der erfindungsgemäßen Zusammensetzung getrunken.

### Kurze Beschreibung der Figuren

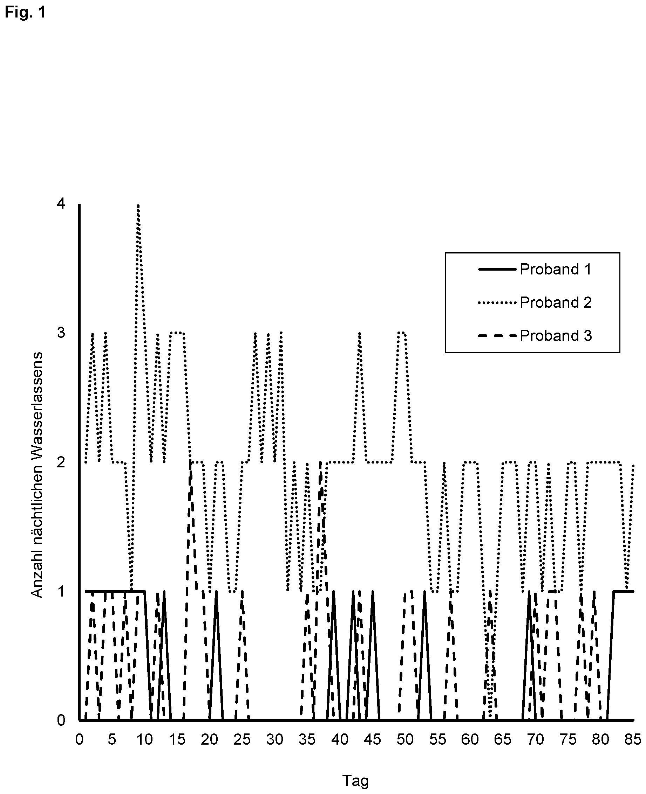

Fig. 1 zeigt die Anzahl des nächtlichen Wasserlassens der Probanden 1 bis 3 während einer 85-tägigen Studie.

### Beschreibung der experimentellen Studie und Ausführungsbeispiele

Um die Wirkung der erfindungsgemäßen Zusammensetzung zu untersuchen, wurde über einen Zeitraum von 85 Tagen eine experimentelle Studie mit drei Probanden durchgeführt. Die Ergebnisse der Studie sind in Fig. 1 als Anzahl des nächtlichen Wasserlassens der Probanden dargestellt. Die Probanden waren zum Zeitpunkt des Studienbeginns älter als 50 Jahre, männlich und von Nykturie betroffen, das heißt sie wachen während der Nachtschlafphase mindestens einmal pro Nacht auf Grund von Harndrang auf und müssen die Blase entleeren. Proband 1 hatte zum Zeitpunkt des Studienbeginns ein Alter von 57 Jahren, Proband 2 eines von 58 Jahren und Proband 3 eines von 57 Jahren.

Die Probanden hatten während der Testphase keinen oder durchschnittlich weniger als 1 g Alkohol pro Tag konsumiert. Die dabei konsumierte Menge an einzelnen Tagen betrug 6-40 g Alkohol.

An Tag 1 bis 10 sowie an Tag 75 bis 85 wurde keine erfindungsgemäße Zusammensetzung eingenommen. Wie Fig. 1 zu entnehmen ist, musste Proband 1 in diesem Zeitraum pro Nacht durchschnittlich einmal die Blase entleeren, Proband 2 durchschnittlich 2,4-mal, Proband 3 hingegen 0,6-mal.

An Tag 11 bis 74 wurden täglich 50 ml der erfindungsgemäßen Zusammensetzung oral eingenommen. Diese umfasste 6,25 g getrocknete Minzeblätter, 10 g frische Minzeblätter, 7 g Blätter grünen Tees, 135 g Ingwer, 2000 ml Wasser und 2000 ml Zitronensaft.

Die Herstellung der erfindungsgemäßen Zusammensetzung wurde wie folgt durchgeführt:
a) Bereitstellen von Minze, grünem Tee, Ingwer, Frucht- oder Gemüsesaft aus einer Frucht- oder Gemüseart oder mehreren Frucht- oder Gemüsearten und gegebenenfalls Wasser;
b) Zerkleinern des Ingwers;
c) Aufkochen des Ingwers in Wasser, Abkühlen und 48 h ziehen lassen;
d) Aufkochen der Mischung aus frischen und getrockneten Minzeblättern, Blättern grünen Tees in Wasser, Abkühlen und 48 h ziehen lassen;
e) Jeweils Abtrennen der Flüssigkeit aus Schritt c) und d) von den Feststoffen;
f) Mischen der Flüssigkeiten aus Schritt c) und d);
g) 4 Tage ziehen lassen der Flüssigkeit aus Schritt f) bei einer Temperatur von 5 °C; und
h) Mischen der Flüssigkeit aus Schritt f) mit Zitronensaft im Verhältnis 1:1.

Wie Fig. 1 zu entnehmen ist, nahm die Anzahl der nächtlichen Blasenentleerungen bei allen Probanden während des Einnahmezeitraums (Tag 11 bis 74) ab. Die Anzahl der Blasenentleerungen bei Proband 1 reduzierte sich während der 65 Tage auf durchschnittlich 0,1-mal pro Nacht, bei Proband 2 auf 1,9-mal und bei Proband 3 auf 0,2-mal.

Ab Tag 75 wurde keine erfindungsgemäße Zusammensetzung mehr eingenommen. Die Anzahl der Blasenentleerungen bei Proband 1 stiegen in diesem Zeitraum von 10 Tagen von 0,1-mal unter Einnahme der erfindungsgemäßen Zusammensetzung auf durchschnittlich 0,4-mal ohne Einnahme der erfindungsgemäßen Zusammensetzung pro Nacht an, war aber im Vergleich zu dem Zeitraum vor Einnahme der erfindungsgemäßen Zusammensetzung reduziert. Bei Proband 2 und Proband 3 blieb die Anzahl der Blasenentleerungen in dem 10-tägigen Zeitraum nach Einnahme der erfindungsgemäßen Erfindung nahezu gleich. Demzufolge waren der nächtliche Harndrang und die damit verbundene Anzahl der nächtlichen Blasenentleerungen bei allen Probanden während der Einnahme der erfindungsgemäßen Zusammensetzung stark reduziert.

Proband 1 zeigte sogar längere beschwerdefreie Zeiträume von 16 Tagen (Tag 22 bis Tag 38) beziehungsweise 14 Tagen (Tag 54 bis 68) und 11 Tagen (Tag 70 bis 81), an denen er keinen nächtlichen Harndrang verspürte, sodass auch keine Blasenentleerung stattfand. Die Probanden berichten zudem über eine angenehmere, leichtere nächtliche Entleerung der Blase.

Die erfindungsgemäße Zusammensetzung bewirkt demnach eine deutliche Verbesserung der Symptome der Nykturie, nämlich der deutlichen Verminderung des nächtlichen Harndrangs.

Im Folgenden werden weitere Ausführungsbeispiele offenbart, die gegebenenfalls nach ihrer Wirkstoffkonzentration und Zitronennote klassifiziert werden. Dabei werden für die Wirkstoffkonzentration die Klassifizierung hoch, mittel, gering und äußerst gering sowie für die Zitronennote die Klassifizierung stark, mittelstark und schwach verwendet. Die Klassifizierung ist in folgender Tabelle definiert.

| | Klasse | Definition |
|---|---|---|
| Wirkstoffkonzentration [Gesamtmasse an getrockneten Minzeblättern, frischen Minzeblättern, Blättern grünen Tees und Ingwer je 1 I Wasser] | hoch | > 150 g bis 1.000 g |
| | mittel | > 100 g bis 150 g |
| | gering | > 50 g bis 100 g |
| | äußerst gering | > 10 g bis 50 g |
| Zitronennote [1 ml Zitronensaft je 1 ml Wasser] | stark | > 0,75 bis 2 |
| | mittelstark | > 0,1 bis 0,75 |
| | schwach | > 0,001 bis 0,1 |

### Ausführungsbeispiel 1:

Die Zusammensetzung gemäß Ausführungsbeispiel 1 umfasst 11,25 g getrocknete Minzeblätter, 10 g frische Minzeblätter, 8,75 g Blätter grünen Tees, 200 g Ingwer, 2000 ml Wasser und 10 ml Zitronensaft.

Diese Zusammensetzung weist eine mittlere Wirkstoffkonzentration und eine schwach ausgeprägte Zitronennote auf.

### Ausführungsbeispiel 2:

Die Zusammensetzung gemäß Ausführungsbeispiel 2 umfasst 6,25 g getrocknete Minzeblätter, 10 g frische Minzeblätter, 7 g Blätter grünen Tees, 135 g Ingwer, 2000 ml Wasser und 2000 ml Zitronensaft.

Diese Zusammensetzung weist eine geringe Wirkstoffkonzentration und eine stark ausgeprägte Zitronennote auf.

### Ausführungsbeispiel 3:

Die Zusammensetzung gemäß Ausführungsbeispiel 3 umfasst 2,25 g getrocknete Minzeblätter, 6 g frische Minzeblätter, 1,75 g Blätter grünen Tees, 40 g Ingwer, 2000 ml Wasser und 500 ml Zitronensaft.

Diese Zusammensetzung weist eine äußerst geringe Wirkstoffkonzentration und eine mittelstark ausgeprägte Zitronennote auf.

### Ausführungsbeispiel 4:

Die Zusammensetzung gemäß Ausführungsbeispiel 4 umfasst 22,5 g getrocknete Minzeblätter, 25 g frische Minzeblätter, 17,5 g Blätter grünen Tees, 400 g Ingwer, 2000 ml Wasser und 750 ml Zitronensaft.

Diese Zusammensetzung weist eine hohe Wirkstoffkonzentration und eine mittelstark ausgeprägte Zitronennote auf.

### Ausführungsbeispiel 5:

Die Zusammensetzung liegt in Form einer Tablette vor. Diese Zusammensetzung umfasst 150 mg Cellulose, 150 mg Fettpulver, 6 mg Ingwer, 1 mg Grüntee, 0,48 mg Minzeblätter und 0,12 mg Minzöl. Eine auf dieser Zusammensetzung basierende Tablette hat ein Gesamtgewicht von 307,6 mg, kann über einen langen Zeitraum aufbewahrt werden und weist eine gute Einnehmbarkeit auf.

Für die Produktion von 1000 Tabletten gemäß Ausführungsbeispiel 5 werden 150 g Cellulose, 150 g Fettpulver, 6 g Ingwer, 1 g Grüntee, 0,48 g Minzeblätter und 0,12 g Minzöl gemischt, gesiebt und mit 300 g einer 1:1 Mischung aus 96 % Ethanol und Wasser granuliert. Anschließend wird das Granulat gesiebt und bei einer Temperatur im Bereich von 40°C bis 50°C getrocknet, bis ein Feuchtegehalt des Granulats von 1,7 % erreicht wird. Das Granulat wird anschließend mit Hilfe einer Tablettiermaschine zu Tabletten verarbeitet. Die Verarbeitung des Granulats zu Tabletten erfolgt in einer Umgebung mit einer maximalen relativen Luftfeuchtigkeit von 40 %.

## Patentansprüche

1. Zusammensetzung zur Verwendung bei der Verringerung von Nykturiesymptomen, umfassend Minze, grünen Tee, Ingwer und gegebenenfalls Frucht- oder Gemüsesaft aus einer Frucht- oder Gemüseart oder mehreren Frucht- oder Gemüsearten und Wasser.

2. Zusammensetzung zur Verwendung bei der Verringerung von Nykturiesymptomen nach Anspruch 1, umfassend getrocknete Minzeblätter, frische Minzeblätter, getrocknete Blätter grünen Tees, frischen Ingwer und Zitrusfruchtsaft aus einer Fruchtart oder mehreren Fruchtarten.

3. Zusammensetzung zur Verwendung bei der Verringerung von Nykturiesymptomen nach Anspruch 2, umfassend
2-25 g getrocknete Minzeblätter;
5-30 g frische Minzeblätter;
1,5-20,0 g getrocknete Blätter grünen Tees;
40-400 g frischen Ingwer;
400-2000 ml Wasser; und
10-2000 ml Zitronensaft.

4. Zusammensetzung zur Verwendung bei der Verringerung von Nykturiesymptomen nach einem der vorhergehenden Ansprüche, ferner 2,5-15,0 g getrockneten Ingwer umfassend.

5. Zusammensetzung zur Verwendung bei der Verringerung von Nykturiesymptomen nach einem der vorhergehenden Ansprüche, ferner 10-200 ml Honig umfassend.

6. Orale Darreichungsform zur Verwendung bei der Verringerung von Nykturiesymptomen umfassend eine Zusammensetzung nach einem der vorhergehenden Ansprüche in Form einer Tablette, einer Kapsel oder eines Granulats.

7. Orale Darreichungsform zur Verwendung bei der Verringerung von Nykturiesymptomen nach Anspruch 6, wobei die Zusammensetzung ferner einen Füllstoff aus der Gruppe von Lactose, Cellulose, Stärke, Saccharose, Polyethylenglykol und Polyethylenoxid oder aus einer Kombination der vorgenannten Stoffe umfasst.

## Claims

1. A composition for use in reducing nocturia symptoms comprising mint, green tea, ginger and optionally fruit or vegetable juice from one or more types of fruit or vegetable and water.

2. A composition for use in reducing nocturia symptoms according to claim 1, comprising dried mint leaves, fresh mint leaves, dried green tea leaves, fresh ginger and citrus fruit juice from one or more types of fruit.

3. A composition for use in reducing nocturia symptoms according to claim 2, comprising
2-25 g of dried mint leaves;
5-30 g of fresh mint leaves;
1.5-20.0 g of dried green tea leaves;
40-400 g fresh ginger;
400-2000 ml water; and
10-2000 ml lemon juice.

4. A composition for use in reducing nocturia symptoms according to any one of the preceding claims, further comprising 2.5-15.0 g of dried ginger.

5. A composition for use in reducing nocturia symptoms according to any one of the preceding claims, further comprising 10-200 ml honey.

6. An oral dosage form for use in reducing nocturia symptoms comprising a composition according to any one of the preceding claims in the form of a tablet, capsule or granules.

7. An oral dosage form for use in reducing nocturia symptoms according to claim 6, wherein the composition further comprises a filler agent selected from the group consisting of lactose, cellulose, starch, sucrose, polyethylene glycol and polyethylene oxide or a combination of the foregoing substances.

## Revendications

1. Une composition destinée à être utilisée pour réduire les symptômes de la nycturie, comprenant de la menthe, du thé vert, du gingembre et éventuellement du jus de fruit ou de légume provenant d'une ou de plusieurs espèces de fruits ou de légumes et de l'eau.

2. La composition destinée à être utilisée pour réduire les symptômes de la nycturie selon la revendication 1, comprenant des feuilles de menthe séchées, des feuilles de menthe fraîche, des feuilles de thé vert séchées, du gingembre frais et du jus d'agrumes provenant d'une ou plusieurs espèces de fruits.

3. La composition destinée à être utilisée pour réduire les symptômes de la nycturie selon la revendication 2, comprenant
2-25 g de feuilles de menthe séchées;
5-30 g de feuilles de menthe fraîche;
1,5-20,0 g de feuilles de thé vert séchées;
40-400 g de gingembre frais;
400-2000 ml d'eau; et
10-2000 ml de jus de citron.

4. La composition destinée à être utilisée pour réduire les symptômes de la nycturie selon l'une quelconque des revendications précédentes, comprenant en outre 2,5 à 15,0 g de gingembre séché.

5. La composition destinée à être utilisée pour réduire les symptômes de la nycturie selon l'une quelconque des revendications précédentes, comprenant en outre 10-200 ml de miel.

6. Une forme galénique orale destinée à être utilisée pour réduire les symptômes de la nycturie, comprenant une composition selon l'une quelconque des revendications précédentes, sous la forme d'un comprimé, d'une gélule ou d'un granulé.

7. La forme galénique orale destinée à être utilisée pour réduire les symptômes de nycturie selon la revendication 6, dans laquelle la composition comprend en outre un agent de remplissage choisi dans le groupe constitué par le lactose, la cellulose, l'amidon, le saccharose, le polyéthylèneglycol et le poly(oxyde d'éthylène) ou une combinaison des substances précitées.
